# EUROPÄISCHE PATENTSCHRIFT

(11) **EP 2 945 554 B1**
(45) Veröffentlichungstag und Bekanntmachung des Hinweises auf die Patenterteilung: **04.03.2020**
(21) Anmeldenummer: 14700904.7
(22) Anmeldetag: 20.01.2014
(51) Int. Cl.: A61B 17/70, A61B 90/00

(54) **IMPLANTATSYSTEM UND BEFESTIGUNGSELEMENT FÜR EIN IMPLANTATSYSTEM**
IMPLANT SYSTEM AND SECURING ELEMENT FOR AN IMPLANT SYSTEM
SYSTÈME D'IMPLANT ET ÉLÉMENT DE FIXATION POUR UN SYSTÈME D'IMPLANT

(30) Priorität: 21.01.2013 DE 102013100574
(43) Veröffentlichungstag der Anmeldung: 25.11.2015
(73) Patentinhaber: Aesculap AG, 78532 Tuttlingen (DE)
(72) Erfinder: KRÜGER, Sven, 78647 Trossingen (DE); HOEFER, Fabian, 78532 Tuttlingen (DE)
(74) Vertreter: Hoeger, Stellrecht & Partner Patentanwälte mbB
(86) Internationale Anmeldenummer: PCT/EP2014/050971
(87) Internationale Veröffentlichungsnummer: WO 2014/111555

(56) Entgegenhaltungen:
- EP-A2- 1 994 902
- WO-A1-2011/109009
- US-A1- 2007 088 357
- US-A1- 2009 182 384

## Beschreibung

Die vorliegende Erfindung betrifft ein Befestigungselement für ein mindestens zwei, an Knochenteilen oder dergleichen festlegbare Befestigungselemente und mindestens ein an den mindestens zwei Befestigungselementen festlegbares Verbindungselement umfassendes Implantatsystem, wobei das Befestigungselement einen distalseitig einen Befestigungsabschnitt aufweisenden Befestigungsteil und ein mit dem Befestigungsteil verbundenes Aufnahmeteil aufweist, wobei das Aufnahmeteil eine Verbindungselementaufnahme zum Aufnehmen des Verbindungselementes und ein Fixierelement zum Festlegen des Verbindungselementes in der Verbindungselementaufnahme umfasst, wobei das Aufnahmeteil aus einem Aufnahmeteilmaterial ausgebildet ist, wobei das Aufnahmeteil mindestens ein Verstärkungselement umfasst, welches aus einem Verstärkungselementmaterial ausgebildet ist, welches eine größere Steifigkeit und/oder Festigkeit aufweist als das Aufnahmeteilmaterial, und wobei das Aufnahmeteil mindestens eine Verstärkungselementaufnahme aufweist, in welche das mindestens eine Verstärkungselement eingesetzt ist.

Ferner betrifft die vorliegende Erfindung ein Implantatsystem mit mindestens zwei an Knochenteilen oder dergleichen festlegbaren Befestigungselementen und mit mindestens einem an den mindestens zwei Befestigungselementen festlegbaren Verbindungselement.

Befestigungselemente der eingangs beschriebenen Art sind insbesondere in Form von Pedikelschrauben als Teile von Implantationssystemen zur Stabilisierung der Wirbelsäule bekannt, die einen gabelförmigen Kopf aufweisen können, in welchen ein Verbindungselement in Form eines Stabes einlegbar und mittels einer Verriegelungsschraube festlegbar ist. Zum Festlegen eines solchen Verbindungselementes sind hohe Kräfte erforderlich, um die gewünschte Stabilität des Implantatsystems nach Implantation zu gewährleisten. Diese Kräfte werden über die Verriegelungsschraube eingeleitet, die sich am Aufnahmeteil abstützt. Ein Problem stellt dabei das aufgrund der hohen Kräfte immer wieder auftretende Aufspreizen des Aufnahmeteils dar, welches insbesondere zu einem Kraftverlust beim Anziehen, einem Verklemmen mit Instrumenten sowie anderen Problemen, und im schlimmsten Fall sogar zum Herausspringen der Verriegelungsschraube aus dem Aufnahmeteil führen kann.

Um das Aufspreizen zu vermeiden, ist es beispielsweise bekannt, zum Festlegen der Verriegelungsschraube Gewinde vorzusehen, die Hinterschnitte aufweisen. Dies ist beispielsweise in der US 6,726,689 B2 beschrieben. Allerdings ist das Ausbilden von Gewinden mit Hinterschnitten kompliziert und insbesondere bei harten Materialien schwierig.

Aus der US 2009/0182384 A1 sind Materialkombinationen für medizintechnische Implantate bekannt. In der EP 1 994 902 A2 ist ein Pedikelschraubenbefestigungssystem beschrieben. Einstellbare Knochenverankerungsanordnungen sind in der US 2007/0088357 A1 offenbart. Die WO 2011/109009 A1 betrifft Knochenschraubenanordnungen aus nichteinheitlichem Material.

Es ist daher einer Aufgabe der vorliegenden Erfindung, die Stabilität des Aufnahmeteils auf möglichst einfache Weise zu verbessern.

Diese Aufgabe wird bei einem Befestigungselement der eingangs beschriebenen Art erfindungsgemäß dadurch gelöst, dass die mindestens eine Verstärkungselementaufnahme verschlossen ist, insbesondere durch Verstemmen eines Bundes, und/oder dass das Befestigungselement mindestens ein Verschlusselement zum Verschließen der mindestens einen Verstärkungselementaufnahme umfasst.

Anstatt das Aufnahmeteil insgesamt aus einem steiferen Material auszubilden, was das Ausbilden spezieller Gewindeformen erschweren würde, bietet die erfindungsgemäß vorgeschlagene Lösung die Option, einfache Gewindeformen zu realisieren und trotzdem die nötige Steifigkeit und/oder Festigkeit des Aufnahmeteils zu gewährleisten, um insbesondere ein Verformen, beispielsweise ein Aufspreizen desselben, zu vermeiden. Es können insbesondere ein, zwei, drei, vier oder auch mehr Verstärkungselemente vorgesehen werden. Um das Anordnen des mindestens einen Verstärkungselements am Aufnahmeteil zu erleichtern, ist es günstig, dass das Aufnahmeteil mindestens eine Verstärkungselementaufnahme aufweist, in welche das mindestens eine Verstärkungselement eingesetzt ist. Beispielsweise kann es sich dabei um eine am Aufnahmeteil seitlich geöffnete Nut handeln, in die das Verstärkungselement eingesetzt und befestigt ist, beispielsweise durch Kleben, Löten oder Schweißen. Denkbar ist es auch, das Verstärkungselement in die Verstärkungselementaufnahme hineinzugießen und so das Aufnahmeteil in einem zweistufigen Gießprozess herzustellen. Auf einfache Weise herstellen lässt sich die mindestens eine Verstärkungselementaufnahme, wenn sie in Form einer Durchbrechung oder eines Sacklochs ausgebildet ist. Vorteilhaft ist es ferner, dass die mindestens eine Verstärkungselementaufnahme verschlossen ist. Beispielsweise kann sie durch Verstemmen eines Bundes verschlossen werden. Durch das Verschließen der Verstärkungselementaufnahme wird beispielsweise das Beschichten des mindestens einen Verstärkungselements, wenn es aus einem Verstärkungselementmaterial mit erhöhtem Allergiepotenzial hergestellt ist, überflüssig, da es durch das Verschließen der mindestens einen Verbindungselementaufnahme quasi mit und in dieser verkapselt wird. Gemäß der Erfindung ist alternativ oder zusätzlich mindestens ein Verschlusselement zum Verschließen der mindestens einen Verstärkungselementaufnahme vorgesehen sein. Mit dem mindestens einen Verschlusselement lässt sich die mindestens eine Verstärkungselementaufnahme auf einfache Weise verschließen. Weist die Verstärkungselementaufnahme zwei oder mehr Öffnungen auf, sind vorzugsweise auch zwei oder mehr Verschlusselemente vorgesehen, die die Verstärkungselementaufnahme vorzugsweise gas- und flüssigkeitsdicht abdichten.

Besonders vorteilhaft ist es, wenn der Elastizitätsmodul des Aufnahmeteilmaterials kleiner ist als der Elastizitätsmodul des Verstärkungselementmaterials. So kann insgesamt mit dem Verstärkungselementmaterial, das einen größeren Elastizitätsmodul aufweist, die gewünschte Steifigkeit und/oder Festigkeit des Aufnahmeteils erreicht werden.

Besonders vorteilhaft ist es, wenn ein Wert des Elastizitätsmoduls des Verstärkungselementmaterials mindestens das 1,5fache des Werts des Elastizitätsmoduls des Aufnahmematerials beträgt. Diese Bedingung ist beispielsweise bei einem Cobalt-Chrom-Stahl als Verstärkungselementmaterial sowie einer TitanLegierung als Aufnahmeteilmaterial erfüllt.

Günstigerweise ist das Aufnahmeteilmaterial röntgentransparent oder im Wesentlichen röntgentransparent. Auf diese Weise können Verschattungen in Röntgenaufnahmen durch das Aufnahmeteil vermieden oder zumindest minimiert werden.

Vorteilhafterweise ist das Verstärkungselementmaterial röntgendicht oder im Wesentlichen röntgendicht. Damit kann das Verstärkungselement genutzt werden, um eine Positionierung des Aufnahmeteils sichtbar zu machen und damit auch eine Position des Befestigungselementes insgesamt in einem Röntgenbild darzustellen.

Vorzugsweise weist das Aufnahmeteilmaterial ein geringeres Allergiepotenzial auf als das Verstärkungselementmaterial. Dies ermöglicht es insbesondere, das Verstärkungselement so am Aufnahmeteil anzuordnen, dass es beispielsweise durch das Aufnahmeteilmaterial vollständig oder im Wesentlichen vollständig umgeben ist, sodass ein Kontakt zwischen dem Verstärkungselementmaterial und dem Körper des Patienten minimiert oder sogar ganz vermieden werden kann.

Besonders vorteilhaft ist es, wenn das Aufnahmeteilmaterial Titan ist oder Titan enthält. Bei Titan handelt es sich um einen bio-inerten Merkstoff, dessen Oxid, also TiO₂ zudem einen bio-aktiven Merkstoff bildet.

Günstig ist es, wenn das Aufnahmeteilmaterial eine Titanlegierung ist, insbesondere Ti6AL4V oder Ti6AL7Nb. Diese Materialien haben sich in der Praxis bereits als Implantatwerkstoffe bewährt.

Bei einer weiteren bevorzugten Ausführungsform der Erfindung kann vorgesehen sein, dass das Verstärkungselementmaterial ein Stahl ist und/oder Cobalt und/oder Chrom enthält und/oder eine Keramik ist oder eine Keramik enthält. Mit derartigen Verstärkungselementmaterialen ist es möglich, Verstärkungselemente mit der erforderlichen Steifigkeit und/oder Festigkeit auszubilden.

Ferner ist es günstig, wenn das Verstärkungselementmaterial eine Cobalt-Chrom-Legierung oder ein Cobalt-Chrom-Stahl ist. Diese Materialien, die zwar ein erhöhtes Allergiepotenzial aufweisen und allergische Reaktionen auslösen können, weisen jedoch eine für die Ausbildung von Implantaten erforderliche Festigkeit auf. Sie können zur Vermeidung allergischer Reaktionen insbesondere in biokompatiblen Materialien gekapselt oder mit biokompatiblen Beschichtungen versehen werden, um insbesondere ein unerwünschtes Austreten von Cobalt und Chrom in den Körper eines Patienten zu vermeiden.

Vorteilhaft ist es, wenn das Aufnahmeteil zur Ausbildung der Verbindungselementaufnahme einen im Wesentlichen U-förmigen Längsschnitt mit zwei freien, in proximaler Richtung vom Befestigungsteil weg weisenden Schenkeln aufweist. Eine solche Ausgestaltung des Aufnahmeteils ermöglicht das Einlegen eines Verbindungselementes von oben. Insgesamt kann so das Aufnahmeteil beispielsweise in Form eines gabelförmigen Kopfes mit einer Befestigungsschraube als Fixierelement ausgebildet werden.

Besonders günstig ist es, wenn das Aufnahmeteil im Bereich der freien Schenkel in Form eines Aufnahmeteilwandabschnitts ausgebildet ist, dessen Dicke im Querschnitt ein relatives oder absolutes Minimum aufweist. Mit anderen Worten bedeutet dies, dass beispielsweise der freie Schenkel zu seinen seitlichen Kanten oder Enden hin dicker sein kann als in etwa in der Mitte zwischen den seitlichen Kanten, wo also die Dicke zumindest ein relatives Minimum, unter Umständen auch ein absolutes Minimum aufweist. Dies ermöglicht es beispielsweise, dass zum Einschrauben einer Fixier- oder Verriegelungsschraube ein entsprechendes Gewinde vorgesehen sein kann, in den Bereichen dickerer Wandstärke jedoch zusätzlich mindestens ein Verstärkungselement angeordnet werden kann, um die gewünschte Stabilität des Aufnahmeteils zu erreichen. An jedem Schenkel können ein, zwei, drei oder auch mehr Verstärkungselemente vorgesehen werden, je nachdem welche Stabilität erforderlich ist und was an Platz an den Schenkeln zur Verfügung steht.

Die Stabilität des Befestigungselementes insgesamt kann erhöht werden, wenn das Befestigungsteil und das Aufnahmeteil unbeweglich miteinander verbunden sind. Beispielsweise könnten sie einstückig ausgebildet, miteinander verlötet oder verschweißt sein, um praktisch jegliche Relativbewegung zwischen dem Befestigungsteil und dem Aufnahmeteil zu unterbinden.

Günstig kann es jedoch auch sein, wenn das Befestigungsteil in einer Justierstellung relativ zum Aufnahmeteil verstellbar und in einer Fixierstellung relativ zum Aufnahmeteil unbeweglich festlegbar ist. Beispielsweise kann das Befestigungselement so in Form einer Polyaxialschraube ausgebildet werden, die es ermöglicht, den Befestigungsteil in einer optimierten Orientierung in einen Knochen einzubringen und dann das Aufnahmeteil relativ zum Befestigungsteil derart auszurichten, dass ein Verbindungselement in optimaler Weise mit gewünschter Ausrichtung eingelegt und am Befestigungselement unbeweglich festgelegt werden kann.

Besonders einfach ausbilden lässt sich ein variabel ausrichtbares Befestigungselement, wenn das Befestigungsteil und das Aufnahmeteil gelenkig miteinander verbunden sind. Denkbar sind hier grundsätzlich alle Arten von Gelenkverbindungen.

Vorteilhaft ist es, wenn eine Gelenkverbindung zwischen einem proximalen Ende des Befestigungsteils und einem distalen Ende des Aufnahmeteils vorgesehen ist und wenn das Aufnahmeteil distalseitig eine Aufnahme für das proximale Ende des Befestigungsteils aufweist. Eine solche Ausgestaltung ermöglicht auch eine gelenkige Kopplung zwischen dem Aufnahmeteil und dem Befestigungsteil.

Um eine nahezu beliebige Ausrichtung des Aufnahmeteils relativ zum Befestigungsteil zu ermöglichen, ist es günstig, wenn die Gelenkverbindung in Form einer Kugelgelenkverbindung ausgebildet ist und wenn das proximale Ende des Befestigungsteils in Form eines kugelsegmentförmigen Kopfes und die Aufnahme in Form eines kalottenförmigen Sitzes ausgebildet ist. Dies gestattet insbesondere eine freie Rotation des Aufnahmeteils relativ zum Befestigungsteil um einen Mittelpunkt des Kopfes.

Das Festlegen eines Verbindungselementes im beziehungsweise am Aufnahmeteil kann weiter vereinfacht werden, wenn das Aufnahmeteil ein mit einem Fixierelementgewinde des Fixierelements zusammenwirkendes Fixiergewinde umfasst.

Günstigerweise ist das Fixiergewinde im Bereich der freien Schenkel ausgebildet. Dies ermöglicht es, dass ein Verbindungselement seitlich aus dem Aufnahmeteil heraussteht, wenn es mit einem Fixierelement, das in das Aufnahmeteil von oben eingeschraubt ist, an diesem festgelegt ist.

Die Stabilität des Befestigungselementes kann insbesondere dadurch erhöht werden, dass das Aufnahmeteil ohne das mindestens eine Verstärkungselement einstückig ausgebildet ist. Beispielsweise kann das Aufnahmeteil so in einem Gießverfahren hergestellt werden.

Gemäß einer weiteren bevorzugten Ausführungsform der Erfindung kann vorgesehen sein, dass das mindestens eine Verstärkungselement kraft- und/oder formschlüssig in der mindestens einen Verstärkungselementaufnahme festgelegt ist. Auf diese Weise kann verhindert werden, dass sich das Verstärkungselement relativ zum Aufnahmeteil bewegen kann, insbesondere aus dieser in unerwünschter Weise austreten kann.

Auf besonders einfache und sichere Weise lässt sich das mindestens eine Verstärkungselement am Aufnahmeteil festlegen, wenn es durch Ausbildung eines Presssitzes in der mindestens einen Verstärkungselementaufnahme gehalten ist. So kann das Verstärkungselement mit einem geringeren Übermaß hergestellt und in die am Aufnahmeteil vorgesehene Verstärkungselementaufnahme eingepresst werden.

Auf einfache Weise herstellen lässt sich die mindestens eine Verstärkungselementaufnahme, wenn sie in Form einer Durchbrechung oder eines Sacklochs ausgebildet ist.

Günstigerweise ist die mindestens eine Verstärkungselementaufnahme in Form einer Bohrung ausgebildet. Eine Bohrung lässt sich einfach herstellen, und zwar sowohl als Durchgangsbohrung als auch als Sacklochbohrung.

Die Herstellung des Befestigungselementes lässt sich weiter vereinfachen, wenn das mindestens eine Verstärkungselement stabförmig oder im Wesentlichen stabförmig ausgebildet ist. Beispielsweise können so Verstärkungselemente durch Abschneiden von Stücken eines Strang- oder Profilmaterials hergestellt werden. Die Querschnittsform des Verstärkungselements kann dabei grundsätzlich beliebig gewählt werden. Vorteilhafterweise weist das Verstärkungselement eine Querschnittsform auf, die beispielsweise im Vergleich zu einem Rundstabprofil eine erhöhte Biegesteifigkeit aufweist, also praktisch ein Verstärkungselement in Form eines biegesteifen Profils.

Vorzugsweise weist das mindestens eine Verstärkungselement einen kreisförmigen oder im Wesentlichen kreisförmigen Querschnitt auf. Ein solches Verstärkungselement lässt sich einfach und sicher formschlüssig in eine in Form einer Bohrung ausgebildete Verstärkungselementaufnahme einsetzen, beispielsweise auch einpressen, wenn ein Durchmesser des Verstärkungselements nur wenig größer ist als ein Innendurchmesser der Verstärkungselementaufnahme.

Gemäß einer weiteren bevorzugten Ausführungsform der Erfindung kann vorgesehen sein, dass die mindestens eine Verstärkungselementaufnahme eine Längsachse definiert, welche parallel oder im Wesentlichen parallel zu einer vom Aufnahmeteil definierten Längsachse und/oder zu einer vom Befestigungsteil definierten Längsachse ausgerichtet ist. Beispielsweise lassen sich so Aufnahmeteilwandabschnitte mit minimaler Dicke ausbilden, wobei trotzdem sichergestellt werden kann, dass die Verstärkungselemente vollständig im Aufnahmeteil aufgenommen werden können. Außerdem hat diese Anordnung den Vorteil, dass beispielsweise in einem Röntgenbild bei Verwendung eines röntgendichten Verstärkungselementmaterials die Orientierung des Aufnahmeteils und/oder des Befestigungsteils erkennbar ist.

Günstig ist es, wenn die mindestens eine Verstärkungselementaufnahme in den freien Schenkeln im Bereich derselben oder an diese angrenzend ausgebildet ist. Dies ermöglicht es, dass das mindestens eine in die mindestens eine Verstärkungselementaufnahme eingesetzte Verstärkungselement die Stabilität des Aufnahmeteils genau dort erhöhen kann, wo sie benötigt wird, nämlich beispielsweise im Bereich der freien Schenkel.

Um eine Beschädigung des Aufnahmeteils zu vermeiden, insbesondere im Bereich der freien Schenkel, ist es vorteilhaft, wenn die mindestens eine Verstärkungselementaufnahme in einem Bereich der freien Schenkel angeordnet oder ausgebildet ist, wo diese eine größere Dicke aufweisen als im Bereich des Minimums der Wandstärke. Mit anderen Worten wird die mindestens eine Verstärkungselementaufnahme vorzugsweise dort ausgebildet, wo mehr Material zur Verfügung steht, um so auch nach Ausbilden der mindestens einen Verstärkungselementaufnahme die Stabilität des Aufnahmeteils an sich nicht zur gefährden, sondern durch Einsetzen des mindestens einen Verstärkungselements in gewünschter Weise zu erhöhen.

Unterschiedliche Dicken im Bereich der freien Schenkel lassen sich auf einfache Weise insbesondere dadurch erreichen, wenn eine im Querschnitt vom Aufnahmeteil definierte Außenkontur unrund ist. Vorzugsweise ist die Außenkontur im Querschnitt oval oder rechteckig, insbesondere rechteckig mit abgerundeten Ecken. So lassen sich freie Schenkel mit variierenden Dicken ausbilden, die für die Ausbildung von Verstärkungselementaufnahmen besonders geeignet sind.

Vorzugsweise bildet das Verstärkungselementmaterial keine äußere Oberfläche des Aufnahmeteils. Dies hat insbesondere den Vorteil, dass als Verstärkungselementmaterial grundsätzlich auch Materialien verwendet werden können, die ein gewisses Allergiepotenzial aufweisen, denn sie können so nicht mit Körpergewebe eines Patienten direkt in Kontakt kommen. Insbesondere kann dies erreicht werden, indem das Verstärkungselementmaterial durch eine geeignete Beschichtung umhüllt oder anderweitig verkapselt oder eingeschlossen wird.

Auf besonders einfache und sichere Weise lässt sich die mindestens eine Verstärkungselementaufnahme verschließen, wenn das Verschlusselement in Form einer kopflosen Schraube oder eines kraft- und/oder formschlüssig in der mindestens einen Verstärkungselementaufnahme festgelegten Deckels ausgebildet ist. So kann zum Verschließen der mindestens einen Verstärkungselementaufnahme die kopflose Schraube eingeschraubt oder der Deckel eingepresst oder beispielsweise mit Klebstoff festgelegt werden.

Zum Festlegen eines Verbindungselementes am Befestigungselement ist es günstig, wenn das Fixiergewinde ein Innengewinde oder ein Außengewinde ist und wenn das Fixierelement in Form einer Schraube oder einer Mutter ausgebildet ist. Insbesondere das Vorsehen eines Innengewindes ermöglicht einen besonders kompakten Aufbau des Befestigungselementes.

Insbesondere ist es vorteilhaft, wenn das Befestigungsteil in Form einer Knochenschraube oder eines Knochenhakens ausgebildet ist. So lässt sich das Befestigungselement mit dem Befestigungsteil sicher in Knochen oder Knochengewebe verankern.

Ferner wird die eingangs gestellte Aufgabe bei einem Implantatsystem der eingangs beschriebenen Art erfindungsgemäß dadurch gelöst, dass mindestens eines der mindestens zwei Befestigungselemente in Form eines der oben beschriebenen, vorteilhaften Befestigungselemente ausgebildet ist.

So lässt sich insgesamt die Stabilität des Implantatsystems insgesamt einfach und sicher erhöhen.

Das Implantatsystem lässt sich an die Physiologie eines Patienten auf einfache Weise individuell anpassen, wenn das Verbindungselement ein Stab oder eine einen stabförmigen Abschnitt aufweisende Platte ist. Abhängig vom verfügbaren Platz sowie der erforderlichen Stabilität kann dann entweder ein gerader oder gekrümmter oder individuell gebogener Stab und/oder auch eine geeignete Platte zur Stabilisierung beispielsweise einer Wirbelsäule eines Patienten genutzt werden.

Die nachfolgende Beschreibung bevorzugter Ausführungsformen der Erfindung dient im Zusammenhang mit den Zeichnungen der näheren Erläuterung. Es zeigen:
- Figur 1:: eine schematische, perspektivische und teilweise durchbrochene Ansicht eines Befestigungsteils eines Implantatsystems; und
- Figur 2:: eine schematische Seitenansicht eines an einer Wirbelsäule festgelegten Implantatsystems mit zwei Befestigungsteilen.

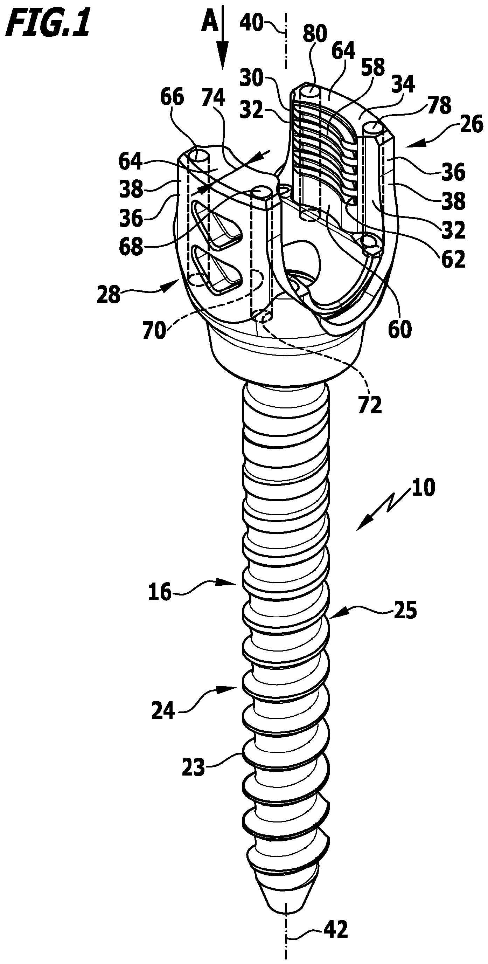

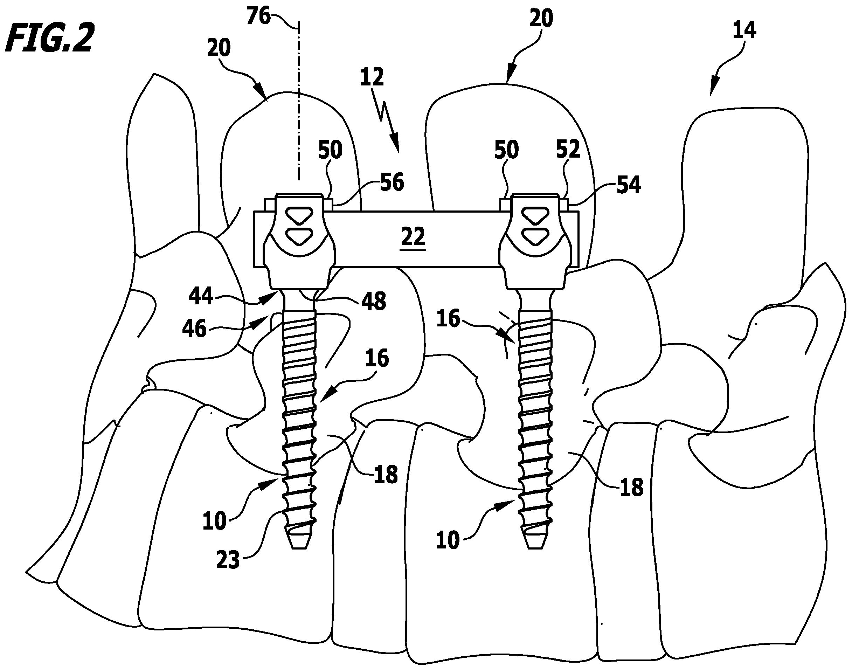

In Figur 1 ist ein Befestigungselement 10 eines in Figur 2 schematisch dargestellten Implantatsystems 12 zur Stabilisierung einer Wirbelsäule 14 schematisch dargestellt. In Figur 2 sind zwei Befestigungselemente 10 in Form von Knochenschrauben 16 dargestellt, die vorzugsweise in Form von Pedikelschrauben ausgebildet sind zum Einschrauben in Pedikel 18 von Wirbeln 20 der Wirbelsäule 14. Das Implantatsystem 12 umfasst ferner ein Verbindungselement 22, welches in Form eines Stabes, und zwar gerade oder gebogen, oder in Form einer einen stabförmigen Abschnitt aufweisenden Platte ausgebildet sein kann.

Das Befestigungselement umfasst einen Befestigungsteil 24 und einen Aufnahmeteil 26, welcher sich proximalseitig an den Befestigungsteil 24 anschließt. Der Befestigungsteil 24 umfasst einen Befestigungsabschnitt 25 mit einem Knochengewinde 23, so dass das Befestigungsteil 24 in Knochengewebe eingeschraubt werden kann. Der Befestigungsteil 24 kann alternativ auch in Form eines Knochenhakens oder eines Knochennagels ausgebildet sein.

Das Aufnahmeteil 26 ist in Form eines gabelförmigen Kopfes 28 ausgebildet, welcher eine Verbindungselementaufnahme 30 aufweist, welche durch zwei einander diametral gegenüber liegende Einschnitte 32 einer in proximaler Richtung weisenden hülsenförmigen Wand 34 des Aufnahmeteils 26 definiert wird. Die Verbindungselementaufnahme 30 wird somit seitlich begrenzt durch zwei einander diametral gegenüberliegende, in proximaler Richtung weisende freie Schenkel 36, die Aufnahmeteilwandabschnitte 38 bilden.

Das Aufnahmeteil 26 definiert eine Längsachse 40, welche bei einem Befestigungselement 10, dessen Aufnahmeteil 26 unbeweglich mit dem Befestigungsteil 24 verbunden ist, mit einer Längsachse 42 des Befestigungsteils 24 fluchtend ausgerichtet ist. Der Aufnahmeteil 26 kann wahlweise auch gelenkig mit dem Befestigungsteil 24 ausgebildet sein, wofür eine Gelenkverbindung 44 vorgesehen sein kann, beispielsweise in Form einer Kugelgelenkverbindung. In diesem Fall ist ein proximales Ende 48 des Befestigungsteils 24 in Form eines kugelsegmentförmigen Kopfes ausgebildet, welcher korrespondierend zu einer Aufnahme in Form eines kalottenförmigen Sitzes im Bereich eines distalen Endes des Aufnahmeteils 26 ausgebildet ist.

Zum Festlegen des Verbindungselementes 22 in der Verbindungselementaufnahme 30 dient ein Fixierelement 50, welches beispielsweise in Form einer kopflosen Schraube 52 mit einem Fixierelementgewinde 54 in Form eines Außengewindes 56 ausgebildet sein kann. Das Fixierelementgewinde 54 ist korrespondierend ausgebildet zu einem Fixiergewinde 58 des Aufnahmeteils 26. Das Fixiergewinde 58 ist ausgebildet an aufeinander zu weisenden Innenflächen 60 der Aufnahmeteilwandabschnitte 38, sodass jeder dieser Aufnahmeteilwandabschnitte 38 eine Mehrzahl von sich über einen Winkelbereich von etwa 80° erstreckenden Gewindenuten 62 aufweist.

Beim Einschrauben des Fixierelements 50 in den Aufnahmeteil 26 zum klemmenden Festlegen des Verbindungselementes 22 am jeweiligen Befestigungselement 10 kann es bei herkömmlichen Schrauben zu einem unerwünschten Aufspreizen der freien Schenkel 36 kommen, wobei sich dabei deren freie Enden 64 in radialer Richtung etwas von der Längsachse 40 weg bewegen. Um dies zu vermeiden, sind an jedem Aufnahmeteilwandabschnitt 38 zwei parallel zueinander und parallel zur Längsachse 40 ausgerichtete Verstärkungselementaufnahmen 66 ausgebildet zum Aufnehmen jeweils eines Verstärkungselements 68. Die Verstärkungselementaufnahme 66 kann wahlweise in Form einer Durchbrechung oder eines Sacklochs 70 ausgebildet sein. Hierfür kann sie insbesondere als Bohrung 72 hergestellt werden. In jede Verstärkungselementaufnahme 66 ist ein Verstärkungselement 68 eingesetzt. Dieses ist stabförmig ausgebildet und weist vorzugsweise einen kreisförmigen oder im Wesentlichen kreisförmigen Querschnitt auf. Bei dem in Figur 1 schematisch dargestellten Ausführungsbeispiel des Befestigungselementes 10 ist das Verstärkungselement 68 in Form eines zylindrischen Stabs ausgebildet.

Die Verstärkungselementaufnahmen 66 sind somit in den freien Schenkeln 36 beziehungsweise im Bereich derselben ausgebildet. Sie sind ferner in einem Bereich der Aufnahmeteilwandabschnitte 38 angeordnet, welche eine in radialer Richtung definierte Dicke 74 aufweisen, die größer ist als im Bereich einer der Längsachse 40 enthaltenden Symmetrieebene 76 des Aufnahmeteils 26. In diesem Bereich weist die Dicke 74 der Aufnahmeteilwandabschnitte ein relatives Minimum auf.

Die Verstärkungselemente 68 sind kraft- und/oder formschlüssig in die Verstärkungselementaufnahmen 66 eingebracht, sodass ein unabsichtliches Herausfallen derselben verhindert wird. Um einen Kontakt der Verstärkungselemente 68 mit Körpergewebe des Patienten zu vermeiden, ist jede Verstärkungselementaufnahme 66 mit einem Verschlusselement 78 verschlossen, sodass die Verstärkungselemente 68 praktisch in den Aufnahmeteilwandabschnitten 38 eingekapselt sind. Das Verschlusselement 78 bildet so praktisch einen Deckel 80 für die Verstärkungselementaufnahme 66. Weist diese mehr als eine Öffnung auf, ist vorzugsweise eine entsprechende Anzahl an Deckeln 80 vorgesehen. Alternativ können die Verstärkungselemente 68 auch mit einer Beschichtung versehen sein, sodass diese bereits vor dem Einsetzen in die Verstärkungselementaufnahmen 66 eingekapselt sind.

Zur Optimierung einer Funktion der Verstärkungselemente 68, nämlich zum Verstärken beziehungsweise Versteifen der Aufnahmeteilwandabschnitte 38, sind die Verstärkungselemente 68 aus einem Verstärkungselementmaterial ausgebildet, welches eine größere Steifigkeit und/oder Festigkeit aufweist als ein Aufnahmeteilmaterial, aus welchen das Aufnahmeteil 26 ausgebildet ist. Vorzugsweise ist der Elastizitätsmodul des Verstärkungselementmaterials größer als der Elastizitätsmodul des Aufnahmeteilmaterials. Insbesondere ist es vorteilhaft, wenn ein Wert des Elastizitätsmoduls des Verstärkungselementmaterials mindestens das 1,5fache des Werts des Elastizitätsmoduls des Aufnahmeteilmaterials beträgt.

Bevorzugt werden zur Ausbildung der Aufnahmeteile 26 der Befestigungselemente 10 Aufnahmeteilmaterialien verwendet, die röntgentransparent oder im Wesentlichen röntgentransparent sind. Im Gegensatz hierzu kann das Verstärkungselementmaterial insbesondere auch röntgendicht oder im Wesentlichen röntgendicht sein. Somit können in einer Röntgenaufnahme die Verstärkungselemente 68 sichtbar gemacht und damit auch eine Ausrichtung des Aufnahmeteils 26 dargestellt werden.

Die beschriebene Verkapselung der Verstärkungselemente 68 beziehungsweise eine Beschichtung derselben ist insbesondere dann vorteilhaft, wenn das Verstärkungselementmaterial ein gewisses Allergiepotenzial aufweist. So kann ein Kontakt des das Allergiepotenzial aufweisenden Materials mit Körpergewebe des Patienten vermieden werden.

Günstig ist es, als Aufnahmeteilmaterial Titan zu verwenden. Es kann jedoch neben Titan auch weitere Elemente enthalten, beispielsweise wenn es in Form einer Titanlegierung ausgebildet ist, beispielsweise Ti6AL4V oder Ti6AL7Nb.

Als Verstärkungselementmaterial eignen sich insbesondere Stähle, beispielsweise Cobalt und Chrom enthaltenden Stähle, wie insbesondere die bei Medizinprodukten häufig verwendete Cobalt-Chrom-Basislegierung.

Die beschriebene Konstruktion des Befestigungselementes 10 ermöglicht es, ein Aufnahmeteilmaterial zu verwenden, welches relativ weich ist, sodass das Fixiergewinde 58 auf einfache Weise in die freien Schenkel 36 eingearbeitet werden kann. Um eine Verformung der freien Schenkel 36 beziehungsweise ein Aufspreizen beim Einschrauben des Fixierelements 50 zu vermeiden, dienen die Verstärkungselemente 68, die dem Aufnahmeteil 26 eine Steifigkeit und/oder eine Festigkeit verleihen, wie sie praktisch nur erreichbar wäre, wenn als Aufnahmeteilmaterial ebenfalls ein Material verwendet wird, aus welchem die Verstärkungselemente 68 hergestellt werden.

Um ausreichend Material an den freien Schenkeln 36 bereitzustellen die das Ausbilden der Verstärkungselementaufnahme 66 ermöglichen, weist das Aufnahmeteil 26 in Draufsicht in distaler Richtung, also in Richtung des Pfeils A in Figur 1, eine im Querschnitt unrunde Außenkontur auf, insbesondere eine ovale beziehungsweise eine rechteckige, vorzugsweise eine rechteckige Außenkontur mit abgerundeten Ecken.

## Patentansprüche

1. Befestigungselement (10) für ein mindestens zwei, an Knochenteilen (20) oder dergleichen festlegbare Befestigungselemente (10) und mindestens ein an den mindestens zwei Befestigungselementen (10) festlegbares Verbindungselement (22) umfassendes Implantatsystem (12), wobei das Befestigungselement (10) einen distalseitig einen Befestigungsabschnitt (25) aufweisenden Befestigungsteil (24) und ein mit dem Befestigungsteil (24) verbundenes Aufnahmeteil (26) aufweist, wobei das Aufnahmeteil (26) eine Verbindungselementaufnahme (30) zum Aufnehmen des Verbindungselementes (22) und ein Fixierelement (50) zum Festlegen des Verbindungselementes (22) in der Verbindungselementaufnahme (30) umfasst, wobei das Aufnahmeteil (26) aus einem Aufnahmeteilmaterial ausgebildet ist, wobei das Aufnahmeteil (26) mindestens ein Verstärkungselement (68) umfasst, welches aus einem Verstärkungselementmaterial ausgebildet ist, welches eine größere Steifigkeit und/ oder Festigkeit aufweist als das Aufnahmeteilmaterial, wobei das Aufnahmeteil (26) mindestens eine Verstärkungselementaufnahme (66) aufweist, in welche das mindestens eine Verstärkungselement (68) eingesetzt ist, **dadurch gekennzeichnet, dass** die mindestens eine Verstärkungselementaufnahme (66) verschlossen ist, insbesondere durch Verstemmen eines Bundes, und/oder dass das Befestigungselement mindestens ein Verschlusselement (78) zum Verschließen der mindestens einen Verstärkungselementaufnahme (66) umfasst.

2. Befestigungselement nach Anspruch 1, **dadurch gekennzeichnet, dass** ein Elastizitätsmodul des Aufnahmeteilmaterials kleiner ist als ein Elastizitätsmodul des Verstärkungselementmaterials.

3. Befestigungselement nach einem der voranstehenden Ansprüche, **dadurch gekennzeichnet, dass** das Aufnahmeteilmaterial röntgentransparent oder im Wesentlichen röntgentransparent ist
und/oder
dass das Verstärkungselementmaterial röntgendicht oder im Wesentlichen röntgendicht ist
und/oder
dass das Aufnahmeteilmaterial ein geringeres Allergiepotenzial als das Verstärkungselementmaterial aufweist
und/oder
dass das Aufnahmeteilmaterial Titan ist oder Titan enthält.

4. Befestigungselement nach einem der voranstehenden Ansprüche, **dadurch gekennzeichnet, dass** das Verstärkungselementmaterial ein Stahl ist und/oder Cobalt und/oder Chrom enthält und/oder eine Keramik ist oder eine Keramik enthält.

5. Befestigungselement nach einem der voranstehenden Ansprüche, **dadurch gekennzeichnet, dass** das Aufnahmeteil (26) zur Ausbildung der Verbindungselementaufnahme (30) einen im Wesentlichen U-förmigen Längsschnitt mit zwei freien, in proximaler Richtung vom Befestigungsteil (24) weg weisenden Schenkeln (36) aufweist,
wobei insbesondere das Aufnahmeteil (26) im Bereich der freien Schenkel (36) in Form eines Aufnahmeteilwandabschnitts (38) ausgebildet ist, dessen Dicke (74) im Querschnitt ein relatives oder absolutes Minimum aufweist.

6. Befestigungselement nach einem der voranstehenden Ansprüche, **dadurch gekennzeichnet, dass** das Befestigungsteil (24) und das Aufnahmeteil (26) unbeweglich miteinander verbunden sind.

7. Befestigungselement nach einem der Ansprüche 1 bis 5, **dadurch gekennzeichnet, dass** das Befestigungsteil (24) in einer Justierstellung relativ zum Aufnahmeteil (26) verstellbar und in einer Fixierstellung relativ zum Aufnahmeteil (26) festlegbar ist.

8. Befestigungselement nach Anspruch 7, **dadurch gekennzeichnet, dass** das Befestigungsteil (24) und das Aufnahmeteil (26) gelenkig miteinander verbunden sind,
wobei insbesondere eine Gelenkverbindung (44) zwischen einem proximalen Ende (48) des Befestigungsteils (24) und einem distalen Ende des Aufnahmeteils (26) vorgesehen ist und das Aufnahmeteil (26) distalseitig eine Aufnahme für das proximale Ende (48) des Befestigungsteils (24) aufweist,
wobei weiter insbesondere die Gelenkverbindung (44) in Form einer Kugelgelenkverbindung (46) ausgebildet ist und wobei das proximale Ende (48) des Befestigungsteils (24) in Form eines kugelsegmentförmigen Kopfes und die Aufnahme in Form eines kalottenförmigen Sitzes ausgebildet ist.

9. Befestigungselement nach einem der voranstehenden Ansprüche, **dadurch gekennzeichnet, dass**
a) das Aufnahmeteil (26) ein mit einem Fixierelementgewinde (58) des Fixierelements (50) zusammenwirkendes Fixiergewinde (54) umfasst,
wobei insbesondere
das Fixiergewinde (54) im Bereich der freien Schenkel (26) ausgebildet ist
und/oder
das Fixiergewinde (58) ein Innengewinde oder ein Außengewinde ist und dass das Fixierelement (50) in Form einer Schraube oder einer Mutter ausgebildet ist,
und/oder
b) das Aufnahmeteil (26) ohne das mindestens eine Verstärkungselement (68) einstückig ausgebildet ist.

10. Befestigungselement nach einem der voranstehenden Ansprüche, **dadurch gekennzeichnet, dass**
a) das Aufnahmeteil (26) mindestens eine Verstärkungselementaufnahme (66) aufweist, in welche das mindestens eine Verstärkungselement (68) eingesetzt ist,
und/oder
b) das mindestens eine Verstärkungselement (68) stabförmig oder im Wesentlichen stabförmig ausgebildet ist.

11. Befestigungselement nach einem der voranstehenden Ansprüche, **dadurch gekennzeichnet, dass** das mindestens eine Verstärkungselement (68) einen kreisförmigen oder im Wesentlichen kreisförmigen Querschnitt aufweist.

12. Befestigungselement nach einem der Ansprüche 9 bis 11, **dadurch gekennzeichnet, dass** die mindestens eine Verstärkungselementaufnahme (66) eine Längsachse definiert, welche parallel oder im Wesentlichen parallel zu einer vom Aufnahmeteil (26) definierten Längsachse (40) und/ oder zu einer vom Befestigungsteil (24) definierten Längsachse (42) ausgerichtet ist
und/oder
dass die mindestens eine Verstärkungselementaufnahme (66) in den freien Schenkeln (36) oder im Bereich derselben oder an diese angrenzend ausgebildet ist
und/oder
dass die mindestens eine Verstärkungselementaufnahme (66) in einem Bereich der freien Schenkel (36) angeordnet oder ausgebildet ist, wo diese eine größere Dicke (74) aufweisen als im Bereich des Minimums der Wandstärke.

13. Befestigungselement nach einem der voranstehenden Ansprüche, **dadurch gekennzeichnet, dass** eine im Querschnitt vom Aufnahmeteil (26) definierte Außenkontur unrund ist, insbesondere oval oder rechteckig, vorzugsweise mit abgerundeten Ecken
und/oder
dass das Verstärkungselementmaterial keine äußere Oberfläche des Aufnahmeteils (26) bildet.

14. Befestigungselement nach einem der voranstehenden Ansprüche,
**dadurch gekennzeichnet, dass**
a) das mindestens eine Verschlusselement (66) in Form einer kopflosen Schraube oder eines kraft- und/oder formschlüssig in der mindestens einen Verstärkungselementaufnahme (68) festgelegten Deckels (80) ausgebildet ist
und/oder
b) das Befestigungsteil (24) in Form einer Knochenschraube oder eines Knochenhakens ausgebildet ist.

15. Implantatsystem (12) mit mindestens zwei an Knochenteilen (20) oder dergleichen festlegbaren Befestigungselementen (10) und mit mindestens einem an den mindestens zwei Befestigungselementen (10) festlegbaren Verbindungselement (22), **dadurch gekennzeichnet, dass** mindestens eines der mindestens zwei Befestigungselemente (10) ein Befestigungselement (10) nach einem der voranstehenden Ansprüche ist, wobei insbesondere das Verbindungselement (22) ein Stab oder eine einen stabförmigen Abschnitt aufweisende Platte ist.

## Claims

1. Fixing element (10) for an implant system (12) comprising at least two fixing elements (10) that are fixable to bone parts (20) or the like and at least one connecting element (22) that is fixable to the at least two fixing elements (10), wherein the fixing element (10) comprises a distal end fixing part (24) comprising a fixing section (25) and a seating part (26) which is connected to the fixing part (24), wherein the seating part (26) comprises a connecting element seating (30) for accommodating the connecting element (22) and a fixing element (50) for fixing the connecting element (22) in the connecting element seating (30), wherein the seating part (26) is formed from a seating part material, wherein the seating part (26) comprises at least one reinforcement element (68) which is formed from a reinforcement element material that has a greater stiffness and/or strength than the seating part material, wherein the seating part (26) comprises at least one reinforcement element seating (66) into which the at least one reinforcement element (68) is inserted, **characterized in that** the at least one reinforcement element seating (66) is sealed off, particularly by caulking a flange, and/or **in that** the fixing element comprises at least one closure element (78) for sealing off the at least one reinforcement element seating (66).

2. Fixing element in accordance with Claim 1, **characterized in that** a modulus of elasticity of the seating part material is smaller than a modulus of elasticity of the reinforcement element material.

3. Fixing element in accordance with any of the preceding Claims, **characterized in that** the seating part material is transparent to X-rays or is substantially transparent to X-rays
and/or
**in that** the reinforcement element material is opaque to X-rays or is substantially opaque to X-rays
and/or
**in that** the seating part material has a lower allergy potential than the reinforcement element material
and/or
**in that** the seating part material is titanium or contains titanium.

4. Fixing element in accordance with any of the preceding Claims, **characterized in that** the reinforcement element material is a steel and/or contains cobalt and/or chromium and/or is a ceramic or contains a ceramic.

5. Fixing element in accordance with any of the preceding Claims, **characterized in that**, for the purposes of forming the connecting element seating (30), the seating part (26) has a substantially U-shaped longitudinal section with two free limbs (36) extending away from the fixing part (24) in the proximal direction,
wherein, in particular, the seating part (26) is in the form of a seating part wall section (38) in the region of the free limbs (36) and the thickness (74) thereof in cross section is at a relative or absolute minimum.

6. Fixing element in accordance with any of the preceding Claims, **characterized in that** that fixing part (24) and the seating part (26) are connected immovably to one another.

7. Fixing element in accordance with any of the Claims 1 to 5, **characterized in that** the fixing part (24) is adjustable relative to the seating part (26) in an adjusting position and is fixable relative to the seating part (26) in a fixing position.

8. Fixing element in accordance with any of the preceding Claims, **characterized in that** the fixing part (24) and the seating part (26) are connected to one another in articulated manner,
wherein, in particular, an articulated joint (44) is provided between a proximal end (48) of the fixing part (24) and a distal end of the seating part (26) and the seating part (26) comprises at the distal end thereof a seating for the proximal end (48) of the fixing part (24),
wherein further, in particular, the articulated joint (44) is in the form of a ball joint connection (46) and **in that** the proximal end (48) of the fixing part (24) is in the form of a spherical segment head and the seating is in the form of a dome shaped seat.

9. Fixing element in accordance with any of the preceding Claims, **characterized in that**
a) the seating part (26) comprises a fixing thread (54) which cooperates with a fixing element thread (58) of the fixing element (50),
wherein, in particular,
the fixing thread (54) is formed in the region of the free limbs (26)
and/or
the fixing thread (58) is an internal thread or an external thread and that the fixing element is in the form of a screw or a nut,
and/or
b) the seating part (26) is formed in one piece without the at least one reinforcement element (68).

10. Fixing element in accordance with any of the preceding Claims, **characterized in that**
a) the seating part (26) comprises at least one reinforcement element seating (66) into which the at least one reinforcement element (68) is inserted,
and/or
b) the at least one reinforcement element (68) is in the form of a rod or substantially in the form of a rod.

11. Fixing element in accordance with any of the preceding Claims, **characterized in that** the at least one reinforcement element (68) has a circular or substantially circular cross section.

12. Fixing element in accordance with any of the Claims 9 to 11, **characterized in that** the at least one reinforcement element seating (66) defines a longitudinal axis which is aligned in parallel with or substantially in parallel with a longitudinal axis (40) defined by the seating part (26) and/or with a longitudinal axis (42) defined by the fixing part (24)
and/or
**in that** the at least one reinforcement element seating (66) is formed in the free limbs (36) or in the region thereof or adjacent thereto
and/or
**in that** the at least one reinforcement element seating (66) is arranged or formed in a region of the free limbs (36) that is of greater thickness (74) than in the region of minimum wall thickness.

13. Fixing element in accordance with any of the preceding Claims, **characterized in that** an outer contour defined by the cross section of the seating part (26) is not round, in particular, is oval or rectangular, with preferably rounded-off corners
and/or
**in that** the reinforcement element material does not form an outer surface of the seating part (26).

14. Fixing element in accordance with any of the preceding Claims, **characterized in that**
a) the closure element (66) is in the form of a headless screw or a cover (80) that is fixed in the at least one reinforcement element seating (68) in force-locking and/or positively locking manner
and/or
b) the fixing part (24) is in the form of a bone screw or a bone hook.

15. Implant system (12) comprising at least two fixing elements (10) that are fixable to bone parts (20) or the like and at least one connecting element (22) that is fixable to the at least two fixing elements (10), **characterized in that** at least one of the at least two fixing elements (10) is a fixing element (10) in accordance with any of the preceding Claims
wherein, in particular, the connecting element (22) is a bar or a plate comprising a section in the form of a bar.

## Revendications

1. Elément de fixation (10) pour un système d'implant (12) englobant au moins deux éléments de fixation (10) pouvant être fixés dans des parties osseuses (20) ou analogues, et au moins un élément de liaison (22) pouvant être fixé ou attaché auxdits au moins deux éléments de fixation (10), l'élément de fixation (10) comprenant une partie de fixation (24), qui, du côté distal, comporte un tronçon de fixation (25), et une partie d'accueil (26), qui est reliée à la partie de fixation (24),
élément de fixation
dans lequel la partie d'accueil (26) comporte un logement d'accueil d'élément de liaison (30) destiné à accueillir l'élément de liaison (22), et un élément d'attache (50) pour attacher l'élément de liaison (22) dans le logement d'accueil d'élément de liaison (30), dans lequel la partie d'accueil (26) est réalisée en un matériau de partie d'accueil,
dans lequel la partie d'accueil (26) comporte au moins un élément de renfort (68) réalisé en un matériau d'élément de renfort, qui présente une rigidité et/ou une résistance plus grande que le matériau de partie d'accueil, et
dans lequel la partie d'accueil (26) présente au moins un logement d'accueil d'élément de renfort (66) dans lequel est inséré ledit au moins un élément de renfort (68),
**caractérisé en ce que** ledit au moins un logement d'accueil d'élément de renfort (66) est fermé, notamment par sertissage d'un collet, et/ou **en ce que** l'élément de fixation comprend au moins un élément de fermeture (78) pour fermer ledit au moins un logement d'accueil d'élément de renfort (66).

2. Elément de fixation selon la revendication 1,
**caractérisé en ce qu'**un module d'élasticité du matériau de partie d'accueil est inférieur à un module d'élasticité du matériau d'élément de renfort.

3. Elément de fixation selon l'une des revendications précédentes, **caractérisé en ce que** le matériau de partie d'accueil est transparent à la radiographie aux rayons X ou sensiblement transparent à la radiographie aux rayons X,
et/ou
**en ce que** le matériau d'élément de renfort est imperméable à la radiographie aux rayons X ou sensiblement imperméable à la radiographie aux rayons X, et/ou
**en ce que** le matériau de partie d'accueil présente un potentiel allergène moindre que celui du matériau d'élément de renfort,
et/ou
**en ce que** le matériau de partie d'accueil est du titane ou renferme du titane.

4. Elément de fixation selon l'une des revendications précédentes, **caractérisé en ce que** le matériau d'élément de renfort est un acier, et/ou renferme du cobalt et/ou du chrome, et/ou est une céramique ou renferme une céramique.

5. Elément de fixation selon l'une des revendications précédentes, **caractérisé en ce que** la partie d'accueil (26), pour former le logement d'accueil d'élément de liaison (30), présente une coupe longitudinale sensiblement en forme de U, avec deux ailes libres (36), qui s'écartent de la partie de fixation (24) dans la direction proximale,
la partie d'accueil (26) étant notamment réalisée, dans la zone des ailes libres (36), sous la forme d'un tronçon de paroi de partie d'accueil (38) dont l'épaisseur (74) présente, en section transversale, un minimum relatif ou absolu.

6. Elément de fixation selon l'une des revendications précédentes, **caractérisé en ce que** la partie de fixation (24) et la partie d'accueil (26) sont reliées mutuellement de manière non mobile.

7. Elément de fixation selon l'une des revendications 1 à 5, **caractérisé en ce que** la partie de fixation (24) peut, dans une position d'ajustement, être réglée par rapport à la partie d'accueil (26), et, dans une position de fixation, être immobilisée par rapport à la partie d'accueil (26).

8. Elément de fixation selon la revendication 7,
**caractérisé en ce que** la partie de fixation (24) et la partie d'accueil (26) sont reliées mutuellement de manière articulée,
une liaison articulée (44) étant notamment prévue entre une extrémité proximale (48) de la partie de fixation (24) et une extrémité distale de la partie d'accueil (26), et la partie d'accueil (26) présentant, du côté distal, un élément d'accueil pour l'extrémité proximale (48) de la partie de fixation (24),
la liaison articulée (44) étant par ailleurs notamment réalisée en tant que liaison d'articulation à rotule (46), et l'extrémité proximale (48) de la partie de fixation (24) en tant que tête en forme de segment de sphère et l'élément d'accueil en tant que siège en forme de calotte sphérique.

9. Elément de fixation selon l'une des revendications précédentes, **caractérisé en ce que**
a) la partie d'accueil (26) comporte un filetage d'attache (54) interagissant avec un filetage d'élément d'attache (58) de l'élément d'attache (50),
la configuration étant notamment telle
que le filetage d'attache (54) est réalisé dans la zone des ailes libres (26),
et/ou
que le filetage d'attache (58) est un filetage intérieur ou un filetage extérieur, et que l'élément d'attache (50) est réalisé sous la forme d'une vis ou d'un écrou,
et/ou
b) la partie d'accueil (26) est réalisée d'un seul tenant sans ledit au moins un élément de renfort (68) .

10. Elément de fixation selon l'une des revendications précédentes, **caractérisé en ce que**
a) la partie d'accueil (26) présente au moins un logement d'accueil d'élément de renfort (66), dans lequel est inséré ledit au moins un élément de renfort (68),
et/ou
b) ledit au moins un élément de renfort (68) est réalisé sous la forme d'un barreau ou sensiblement sous la forme d'un barreau.

11. Elément de fixation selon l'une des revendications précédentes, **caractérisé en ce que** ledit au moins un élément de renfort (68) présente une section transversale de forme circulaire ou sensiblement de forme circulaire.

12. Elément de fixation selon l'une des revendications 9 à 11, **caractérisé en ce que** ledit au moins un logement d'accueil d'élément de renfort (66) définit un axe longitudinal, qui est orienté de manière parallèle ou sensiblement de manière parallèle à un axe longitudinal (40) défini par la partie d'accueil (26) et/ou à un axe longitudinal (42) défini par la partie de fixation (24), et/ou
**en ce que** ledit au moins un logement d'accueil d'élément de renfort (66) est réalisé dans les ailes libres (36), ou dans la zone de celles-ci, ou bien encore de manière adjacente à celles-ci,
et/ou
**en ce que** ledit au moins un logement d'accueil d'élément de renfort (66) est agencé ou réalisé dans une zone des ailes libres (36) où celles-ci présentent une épaisseur (74) plus importante que dans la zone du minimum d'épaisseur de paroi.

13. Elément de fixation selon l'une des revendications précédentes, **caractérisé en ce qu'**un contour extérieur défini par la partie d'accueil (26), en section transversale, n'est pas circulaire, et est notamment ovale ou rectangulaire, de préférence avec des coins arrondis,
et/ou
**en ce que** le matériau d'élément de renfort ne forme aucune surface extérieure de la partie d'accueil (26).

14. Elément de fixation selon l'une des revendications précédentes, **caractérisé en ce que**
a) au moins un élément de fermeture (66) est réalisé sous la forme d'une vis sans tête ou d'un couvercle (80) fixé par adhérence et/ou par complémentarité de formes dans ledit au moins un logement d'accueil d'élément de renfort (68),
et/ou
b) la partie de fixation (24) est réalisée sous la forme d'une vis à os ou d'un crochet à os.

15. Système d'implant (12) comprenant au moins deux éléments de fixation (10) pouvant être fixés dans des parties osseuses (20) ou analogues, et comprenant également au moins un élément de liaison (22) pouvant être attaché auxdits au moins deux éléments de fixation (10), **caractérisé en ce que** l'un au moins desdits au moins deux éléments de fixation (10), est un élément de fixation (10) selon l'une des revendications précédentes, l'élément de liaison (22) étant notamment un barreau ou une plaque présentant un tronçon en forme de barreau.
